# EUROPEAN PATENT SPECIFICATION

(11) **EP 1 904 624 B1**
(45) Date of publication and mention of the grant of the patent: **21.09.2011**
(21) Application number: 05761298.8
(22) Date of filing: 20.07.2005
(51) Int. Cl.: C12N 5/0789

(54) **METHOD FOR CULTURING AND PROLIFERATING HEMATOPOIETIC STEM CELLS AND PROGENITOR CELLS USING HUMAN ENDOMETRIAL CELLS**
VERFAHREN ZUR KULTIVIERUNG UND VERMEHRUNG HÄMATOPOETISCHER STAMMZELLEN UND VORLÄUFERZELLEN UNTER VERWENDUNG MENSCHLICHER ENDOMETRIUMZELLEN
PROCÉDÉ POUR CULTIVER ET FAIRE PROLIFÉRER DES CELLULES SOUCHES HÉMATOPOÏÉTIQUES ET DES CELLULES PROGÉNITRICES UTILISANT DES CELLULES ENDOMÉTRIALES HUMAINES

(43) Date of publication of application: 02.04.2008
(73) Proprietor: SEOUL NATIONAL UNIVERSITY INDUSTRY FOUNDATION, Gwanak-gu Seoul 151-050 (KR)
(72) Inventor: KANG, Kyung Sun, Seocho-gu, Seoul 137-935 (KR)
(74) Representative: Hiebl, Inge Elisabeth
(86) International application number: PCT/KR2005/002335
(87) International publication number: WO 2007/011088

(56) References cited:
- WO-A-01/34776
- WO-A-03/070922
- WO-A1-03/006691
- KR-A- 20010 046 513
- KR-A- 20030 069 115
- US-A1- 2004 180 432
- MIRALLES G D ET AL: "CD34+CD38-lin- cord blood cells develop into dendritic cells in human thymic stromal monolayers and thymic nodules." JOURNAL OF IMMUNOLOGY (BALTIMORE, MD. : 1950) 1 APR 1998, vol. 160, no. 7, 1 April 1998 (1998-04-01), pages 3290-3298, XP002557253 ISSN: 0022-1767
- DA SILVA C L ET AL: "A human stromal-based serum-free culture system supports the ex vivo expansion/maintenance of bone marrow and cord blood hematopoietic stem/progenitor cells" EXPERIMENTAL HEMATOLOGY, NEW YORK, NY, US, vol. 33, no. 7, 1 July 2005 (2005-07-01), pages 828-835, XP004936584 ISSN: 0301-472X
- VASSILIEVA S ET AL: "Establishment of SSEA-1- and Oct-4-expressing rat embryonic stem-like cell lines and effects of cytokines of the IL-6 family on clonal growth" EXPERIMENTAL CELL RESEARCH, ACADEMIC PRESS, US, vol. 258, no. 2, 1 August 2000 (2000-08-01), pages 361-373, XP002232587 ISSN: 0014-4827
- HOGAN C.J. ET AL.: 'Differential long-term and multilineage engraftment potential from subfractions of human CD34+ cord blood cells transplanted into NOD/SCID mice' PNAS vol. 99, no. 1, 08 January 2002, pages 413 - 418, XP003007073
- YAMAGUCHI M. ET AL.: 'Serum-free coculture system for ex vivo expansion of human cord blood primitive progenitors and SCID mouse-reconstituting cells using human bone marrow primary stromal cells' EXPERIMENTAL HEMATOLOGY vol. 29, 2001, pages 174 - 182, XP002992704

## Description

### TECHNICAL FIELD

The present invention relates to a method for culturing and proliferating hematopoietic stem cells or progenitor cells, more particularly, to a method for culturing and proliferating hematopoietic stem cells or progenitor cells, in which the hematopoietic stem cells or the progenitor cells are co-cultured with human endometrial stromal cells or epithelial cells so as to promote either the expansion of hematopoietic stem cell-derived CD34⁺ cells or the growth of the progenitor cells.

### BACKGROUND ART

Hematopoietic stem cells and progenitor cells, which are derived from umbilical cord blood and can be differentiated into blood cells, such as erythrocytes, leucocytes and platelets, have the characteristics of stem cells, such as self-renewing capacity, high capacity for cell division, and multi-potential differentiation capacity (van der Kooy, D. et al., Science, 287:1439, 2000).

Currently, transplantation of hematopoietic stem cells or progenitor cells by the use of umbilical cord blood is clinically activated. The hematopoietic stem cells or the progenitor cells are recognized as important sources for transplantation, particularly when cancer patients after a heavy dose of chemotherapy are in malignant hematopoietic conditions, the cells of their own tissue are unsuitable, and adult donors do not exist. By transplantation of such hematopoietic stem cells or progenitor cells, various diseases are-being treated. For example, transplantation of hematopoietic stem cells or progenitor cells has been attempted mainly to treat blood cancer-related diseases, such as acute and chronic leukemia, aplastic anemia, myelodysplastic syndromes, multiple myeloma, malignant lymphoma, and recently, also shows good therapeutic effects on solid cancers, such as breast cancer, ovarian cancer, kidney cancer, and small cell lung cancer; malignant autoimmune diseases, such as refractory systemic erythematous lupus and refractory rheumatoid arthritis; and incurable diseases such as Krabbe disease (Lennard, et al., BMJ, 321:433, 2000; Ikehara, Experimental Hematology, 29:661, 2001).

However, since only a small amount of blood is obtained from umbilical cords, the amount of ultimately obtainable hematopoietic stem cells or progenitor cells is inevitably limited. Transplantation of hematopoietic stem cells or progenitor cells in an unexpanded state is limited mostly to juvenile patients. Thus, for the past decade, an interest in the *in vitro* expansion of hematopoietic stem cells or progenitor cells derived from not only bone marrow but also umbilical cord blood has been increased (Cairo, M.S. et al., Blood, 90:4665, 1997; Mayani, H. et al., Stem Cells, 16:153, 1998).

Accordingly, for the transplantation of hematopoietic stem cells or progenitor cells, the development of technology for obtaining large amounts of desired hematopoietic stem cells or progenitor cells is urgently needed. To expand such hematopoietic stem cells or progenitor cells in large amounts, the development of various methods and optimal culture conditions has been attempted.

Typical culture methods known up to now include a method of culturing the cells *ex vivo* in a medium containing various recombinant stimulatory cytokines (Metcalf, D. et al., Biomed. Pharmacother., 55:75, 2001; Ian McNiece et al., Experimental Hematology, 29:3, 2001), a method of using an antibody to a hematopoietic inhibitor, and a method of using a stromal cell line (Yamaguchi, M. et al., Exp Hematol., 29:174, 2001).

In clinical transplantation of hematopoietic stem cells or progenitor cells, cytokines used in culture of the cells could show various problems. For example, due to their side effects, they could cause the risk of infection or tumor development. For example, the use of cytokine IL-2 causes severe side effects, such as a severe low blood pressure symptom and an acute capillary leak syndrome and thus is inevitably limited. The acute capillary leak syndrome is a phenomenon where tissue fluid from capillary vessels is accumulated in tissue (Rosenstein, M. et al., J. Immunol., 137:1735, 1986). In most studies reported up to now, a fluorescence-activated cell sorter (FACS) was used to isolate CD34⁺ cells, but this isolation technique costs much and requires a lot of time.

Miralles G. D. et al., "CD34+CD38-lin- Cord Blood Cells Develop into Dendritic Cells in Human Thymic Stromal Monolayers and Thymic Nodules", Journal of Immunology (Baltimore, MD.: 1950), April 1, 1998, vol. 160, no. 7, pages 3290 - 3298, discloses the development of primitive human hemopoietic stem cells into mature DCs without cytokine or serum supplementation in the presence of thymic stroma. Coculture of CD34⁺CD38⁻lineage (lin)⁻ and CD34⁺CD38⁺lin⁻ umbilical cord blood cells with thymic stromal monolayers induced 43-fold and 32-fold expansions, respectively, of umbilical cord blood progenitors and also generated large numbers of cells with the morphologic, phenotypic and functional characteristics of mature DCs.

Da Silva C. L. et al., "A human stromal-based serum-free culture system supports the ex vivo expansion/maintenance of bone marrow and cord blood hematopoietic stem/progenitor cells", Experimental Hematology, New York, NY, US, vol. 33, no. 7, July 1, 2005, pages 828 - 835, relates to an investigation of the role of human stromal layers (hu-ST) on the ex vivo expansion/maintenance of human hematopoietic stem/progenitor cells (HSC) from adult bone marrow (BM) and umbilical cord blood (CB). It is disclosed that significant expansion of BM and CB CD34⁺ and CD34⁺CD38⁻ cells were achieved in the presence of hu-ST.

Vassilieva S. et al., "Establishment of SSEA-1- and Oct-4-Expressing Rat Embryonic Stem-like Cell Lines and Effects of Cytokines of the IL-6 Family on Clonal Growth", Experimental Cell Research, Academic Press, US, vol. 258, no. 2, August 1, 2000, pages 361 - 373, shows long-term cultivation of alkaline phosphatase-positive rat embryonic stem-like (RES) cell lines. Rat embryonic stem-like cell lines could be established from rat blastocysts and were able to proliferate as undifferentiated alkaline phosphatase-positive cells.

WO O1/34776 discloses methods of obtaining human hematopoietic cells from human embryonic stem cells using mammalian stromal cells.

Accordingly, the present inventors have made extensive efforts to solve the above-described problems occurring in the prior art, and consequently, found that when CD34⁺ hematopoietic stem cells or progenitor cells are isolated with high purity without the use of the prior fluorescence-activated cell sorter and then the isolated hematopoietic stem cells or progenitor cells are co-cultured with human endometrial stromal cells or epithelial cells without the use of artificial hormones, such as cytokine or serum, the hematopoietic stem cells or the progenitor cells can be effectively expanded in a short period of time, thereby completing the present invention.

### DISCLOSURE OF THE INVENTION

It is a main object of the present invention to provide a method for culturing and proliferating hematopoietic stem cells or progenitor cells, in which CD34⁺ hematopoietic stem cells or progenitor cells are co-cultured with human endometrial stromal cells or epithelial cells without the use of artificial hormones, such as cytokine or serum.

To achieve the above object, the present invention provides a method for culturing and proliferating hematopoietic stem cells or progenitor cells, the method comprising the steps of: (a) culturing hematopoietic stem cells or progenitor cells derived from umbilical cord blood, marrow, peripheral blood or non-human embryonic stem cells; (b) culturing endometrial stromal cells or epithelial cells derived from uterine tissue; and (c) co-culturing the hematopoietic stem cells or progenitor cells cultured in the step (a) and the endometrial stromal cells or epithelial cells cultured in the step (b).

In the present invention, the hematopoietic stem cells or progenitor cells are preferably obtained by the steps of: (a) monolayer culturing, suspension culturing or co-culturing mononuclear or progenitor cells derived from umbilical cord blood, marrow, peripheral blood or non-human embryonic stem cells; (b) reacting the monolayer-, suspension- or co-cultured cells with an antibody to a hematopoietic stem cell- or progenitor cell-specific antigen; and (c) isolating the cells bound to the antibody by means of a cell sorter or a column.

In the present invention, the endometrial stromal cells or epithelial cells are preferably obtained by the steps of: (a) placing and culturing finely cut uterine tissue in a cell culture medium containing collagenase; (b) passing the cultured cells through a cloth sieve with a pore size of 30-50 µm, and collecting the epithelial cells caught on the cloth sieve; and (c) culturing the cells passed through the sieve in the step (b) on a cell culture dish and then isolating the stromal cells adhered to the cell culture dish by washing the cell culture dish with the culture broth.

In the inventive method for culturing and proliferating hematopoietic stem cells or progenitor cells, the antibody to the hematopoietic stem cell-specific antigen is preferably an antibody to CD34⁺, CD38⁻, Thy1.1⁺, lin⁻, CD45⁺, or KDR⁺, and the antibody to the progenitor cell-specific antigen is preferably an antibody to Oct4, Nanog, Wnt, Stat3, alkaline phophatase, or SSEA-1. It is also possible to use an antibody labeled with a fluorescent substance or magnetic microbeads, for example, a CD34⁺ antibody labeled with magnetic microbeads.

In the present invention, the cell sorters preferably a magnetic cell sorter in place of a fluorescence-activated cell sorter, and the column is preferably a magnetic column. Cells obtainable by the above steps are umbilical cord blood-derived CD34⁺ and CD38⁻ cells.

Also, in the inventive method for culturing and proliferating hematopoietic stem cells or progenitor cells, the co-culture is performed for 1-120 hours, and preferably 12-96 hours, in serum-free or serum medium, and preferably serum-free medium.

In another aspect, the present invention provides hematopoietic stem cells cultured and proliferated by the above-described method, in which the hematopoietic stem cells show one or more positive responses to antibodies to CD34, CD133, C-Kit, CD117, CD45 and KDR, and show negative responses to antibodies to CD38 and lin.

In still another aspect, the present invention provides progenitor cells cultured and proliferated by the above-described method, in which the progenitor cells show one or more positive responses to antibodies to Oct4, Nanog, Wnt, Stat3, alkaline phophatase and SSEA-1.

The above and other features and embodiments of the present invention will be more fully apparent from the following detailed description and appended claims.

### BRIEF DESCRIPTION OF DRAWINGS

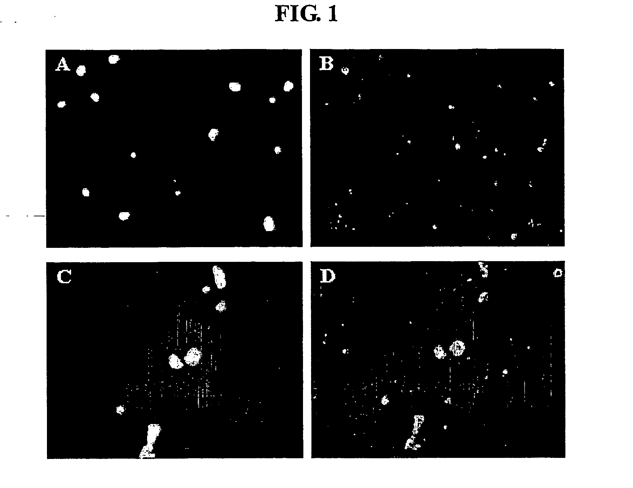

**FIG 1** illustrates photographs showing the results of microscopic observation for CD34⁺ cells quantified by immunocytochemistry after positive selection. In FIG 1, "A" and "B" are photographs viewed at x 100 magnification, and "C" and "D" are photographs viewed at x200 magnification.

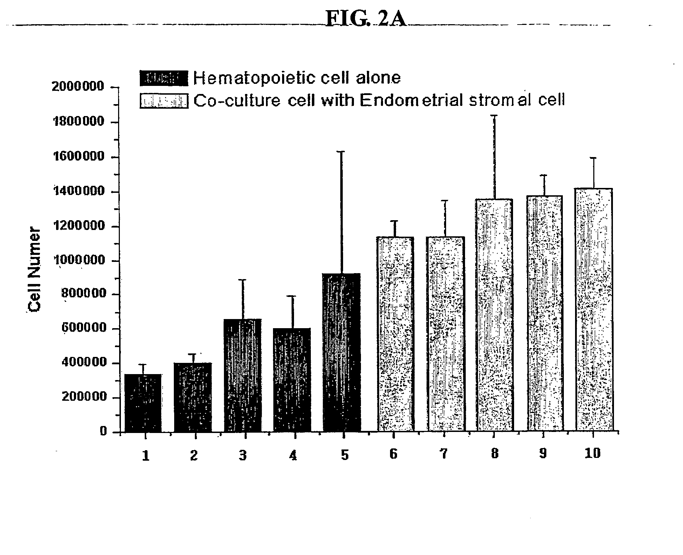

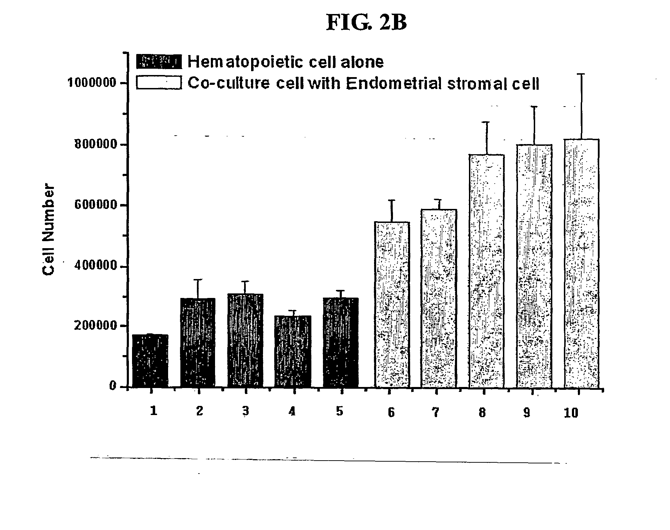

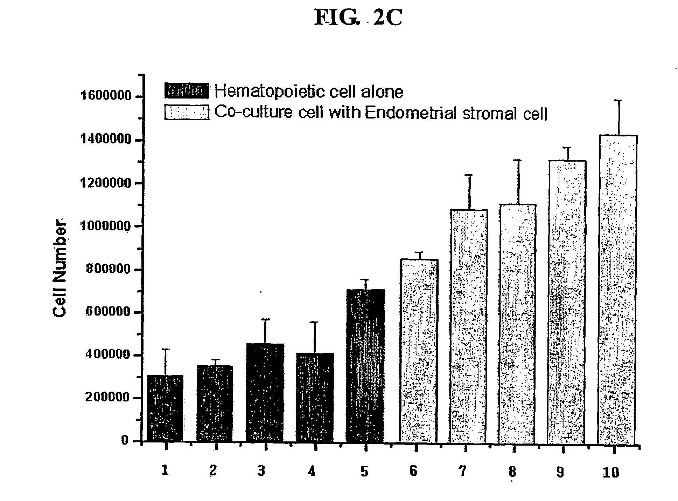

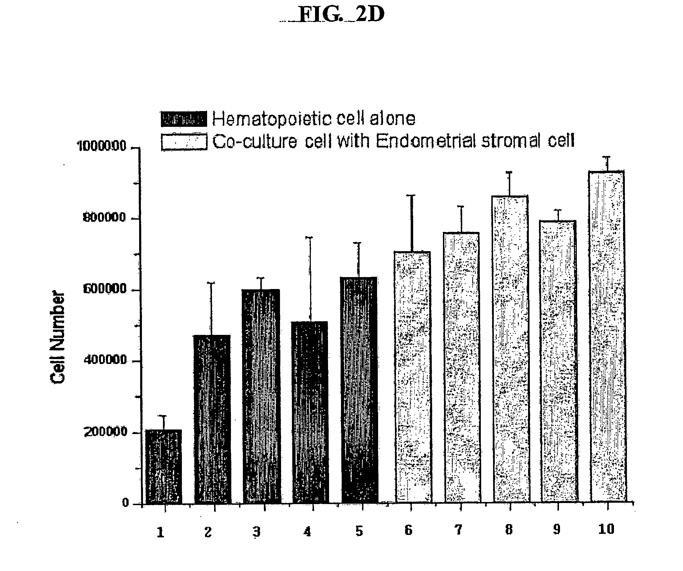

**FIGS. 2A** to **2D** are graphs showing measurement results for cell number at intervals after co-culture of CD34⁺-enriched cells and endometrial stromal cells, followed by treatment with cytokines. **FIGS. 2A-2D** show results after 24 hours, 48 hours, 72 hours and 96 hours, respectively. In **FIGS. 2A-2D****,** graphs 1-5 are results for culture of hematopoietic stem cells alone, and graphs 6-10 are results for the co-culture (1: negative control; 2: EPO-treated group; 3: IL-3-treated group; 4: GM-CSF-treated group; 5: EPO + IL-3 + GM-CSF-treated group; 6: none; 7: EPO-treated group; 8: IL-3-treated group; 9: GM-CSF-treated group; and 10: EPO + IL-3 + GM-CSF-treated group).

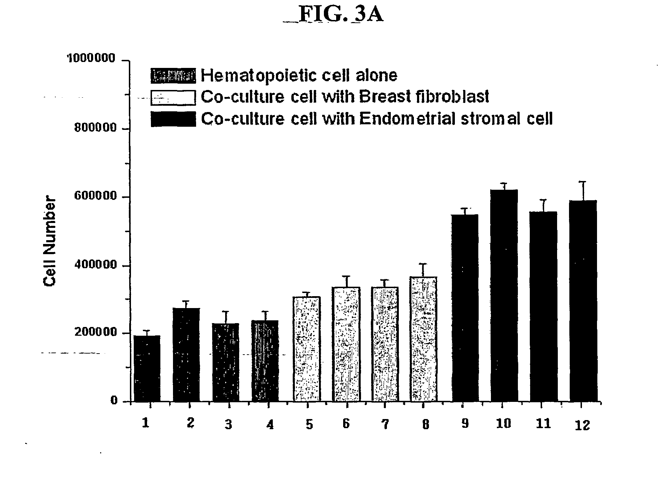

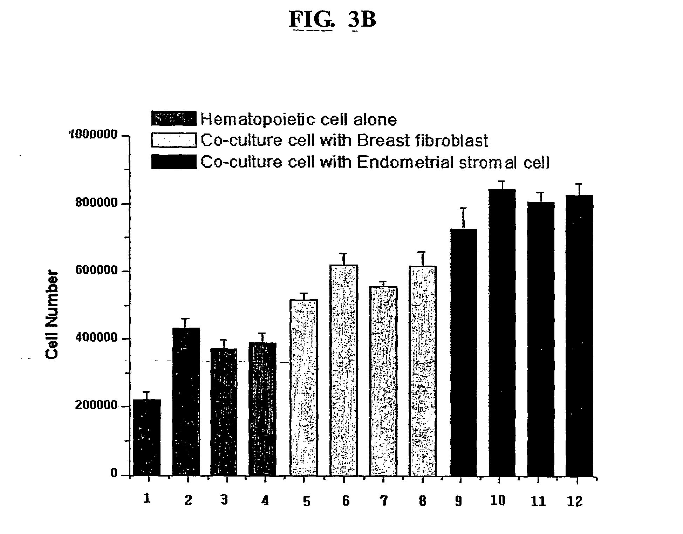

**FIGS. 3A** and **3B** are graphs showing measurement results for cell number at intervals after co-culture of CD34⁺-enriched cells and breast fibroblasts or endometrial stromal cells, followed by treatment with cytokines. **FIGS. 3A** and **3B** show results after 24 hours and 48 hours, respectively. In **FIGS. 3A** and **3B****,** graphs 1-4 are results for culture of the hematopoietic stem cells alone, graphs 5-8 are results for co-culture with the breast fibroblasts, and graphs 9-12 are results for co-culture with the endometrial stromal cells (1: negative control; 2: IL-3-treated group; 3: LIF-treated group; 4: IL-3 + LIF-treated group; 5: none; 6: IL-3-treated group; 7: LIF-treated group; 8: IL-3 + LIF-treated group; 9: none; 10: IL-3-treated group; 11: LIF-treated group; and 12: IL-3 + LIF-treated group).

### DETAILED DESCRIPTION OF THE INVENTION

The present invention relates to a method for culturing and proliferating hematopoietic stem cells or progenitor cells, in which a CD34⁺- and CD38⁻-enriched hematopoietic stem cell population is isolated from umbilical cord blood by an immuno-magnetic technique and expanded by co-culture with endometrial stromal cells or epithelial cells.

As used herein, the term "umbilical cord blood" is defined as blood collected from umbilical veins that connect the placenta to an embryo in mammals, including human beings. In the inventive method for culturing and proliferating hematopoietic stem cells or progenitor cells, human umbilical cord blood is preferably used.

In the cell isolation by the immuno-magnetic technique, at least one antibody selected from antibodies to cell surface antigens expressed in hematopoietic stem cells or progenitor cells can be used. In this regard, the antibodies to the hematopoietic stem cell-specific antigens include antibodies to CD34⁺, CD38⁻, Thy1.1⁺, lin⁻, CD45⁺, or KDR⁺, and the antibodies to the progenitor cell-specific antigens include antibodies to Oct4, Nanog, Wnt, Stat3, alkaline phophatase, or SSEA-1. For the hematopoietic stem cells, an antibody to a CD34⁺ antigen is preferably used.

Furthermore, the antibody labeled with a suitable marker can also be used according to a conventional cell isolation technique. When a magnetic cell sorter or a magnetic column is used in the cell isolation, an antibody labeled with magnetic microbeads is preferably used, and when a FACsorter is used, it is preferable to use an antibody labeled with a fluorescent substance, such as fluorescein isothiocyanate (FITC), phycoerythrin (PE), or PerCP. After the reaction of the cells with an antibody to the cell surface antigen for a given time, cells bound to the corresponding antibody are isolated by a sorter or a column. The isolated cells are hematopoietic stem cells which express umbilical cord blood-derived CD34⁺ and CD38⁻ cells on the surface thereof. In the present invention, the isolated cells were represented as "CD34⁺ cells".

In a preferred embodiment of the present invention, CD34⁺ cells are isolated by immunomagnetic columns using magnetic microbeads other than by the FACsorter, and stabilized on a cell culture medium. The entire process for isolating the cells according to the present invention is preferably performed in an aseptic environment.

The cells isolated by the above-described process have much higher activity than cells isolated by the fluorescence-activated cell sorter do and require low cost and less time for their isolation. This indicates that this process is more suitable for the preparation of hematopoietic stem cells or progenitor cells for clinical transplantation.

As used herein, the term "hematopoietic stem cells or progenitor cells" refers to progenitor cells making the erythrocytes, leukocytes and platelets of blood. The hematopoietic stem cells or progenitor cells play an important role in treating blood diseases, immune diseases, cancers, and the like, and can be isolated from marrow, peripheral blood, umbilical cord blood, non-human embryonic stem cells, and the like. In the present invention the term "progenitor cells" is defined as including the stem cells derived from marrow, umbilical cord blood, non-human embryo, adipose, etc.

The endometrial stromal cells or epithelial cells used in the present invention can be obtained by the primary culture to tenth subculture of the endometrial tissue of mammals, including human beings. The endometrial tissue can preferably be obtained from the uterus taken from the human beings, and more preferably, from the uterus taken from women in the proliferative phase (5-14 days) and the secretory phase (15-26 days), who received no hormonal treatment.

The isolation of the epithelial cells and stromal cells from the endometrial tissue is performed in the following manner. (1) Finely cut uterine tissue is placed in a cell culture medium containing collagenase, followed by culturing at 37 °C for 2-3 hours. (2) The cultured cells are passed through a cloth sieve with a pore size of 100-200 µm. (3) The cells passed through the sieve are passed again through a cloth sieve with a pore size of 30-50 µm. At this time, the epithelial cells are caught on the upper side of the sieve, and the stromal cells are passed through the sieve. (4) The cells passed through the sieve in the step (3) are cultured on a cell culture dish, and after one hour of the culture, the cell culture dish is washed with the culture broth so as to isolate and collect endometrial stromal cells adhered to the cell culture dish.

The umbilical cord blood-derived CD34⁺ and CD38⁻ cells, and endometrial stromal cells or epithelial cells isolated according to the present invention are used in a cell culture medium containing insulin, transferrin, selenium, bovine serum albumin, linoleic acid, and the like. The cell culture medium is any one selected from general cell culture media, such as DMEM, α-MEM, McCoys 5A medium, Eagle's basal medium, CMRL medium, Glasgow minimal essential medium, Ham's F-12 medium, Iscove's modified Dulbecco's medium, Liebovitz' L-15 medium, RPMI 1640 medium, and M199/F12. For the umbilical cord blood-derived CD34⁺ and CD38⁻ cell culture, RPMI1640 is preferably used, and for the epithelial cell culture, M199/F12 is preferably used.

If necessary, the cell culture medium used in the present invention may additionally contain at least one adjuvant component, in addition to the above-described components. Examples of the additional components include equine, bovine or human serum, antibibiotics and antifungal-agents for the prevention of microbial contamination, such as penicillin G streptomycin sulfate, amphotericin B, gentamycin, and nystatin.

In a preferred embodiment of the present invention, the endometrial stromal cells are cultured in a M199/F12 medium containing FBS, insulin, transferrin, selenium, bovine serum albumin, linoleic acid, penicillin, streptomycin and amphotericin B, and the epithelial cells are cultured in a medium which is the same as the above except that it contains BPE in place of FBS.

Moreover, in the inventive method for culturing and proliferating the hematopoietic stem cells or progenitor cells, co-culture of the umbilical cord blood-derived CD34⁺ and CD38⁻ cell population and the endometrial stromal cells or epithelial cells, is preferably performed by seeding and culturing the umbilical cord blood-derived CD34⁺ and CD38⁻ cells in endometrial stromal cells or epithelial cells prepared by culturing the first endometrial stromal cells or epithelial cells obtained above in a serum-free or serum medium. In this regard, the ratio of the endometrial stromal cells to the CD34⁺ cells is preferably 1-5:1.

The co-culture is performed for 1-120 hours and preferably 24-96 hours, without the addition of cytokine or serum for cell transplantation, in which case the umbilical cord blood derived CD34⁺ and CD38⁻ cells are proliferated by the influence of stimulants secreted from the endometrial stromal cells or epithelial cells.

In examples of the present invention, co-culture of hematopoietic stem cells or progenitor cells and human endometrial stromal cells or epithelial cells showed the most excellent effect on the proliferation of the hematopoietic stem cells or progenitor cells, as compared to a negative control group without co-culture, and a positive control group co-cultured with human breast fibroblasts. Also, co-culture of hematopoietic stem cells or progenitor cells and human uterine stromal cells showed stimulated growth or similar growth as compared to the case of the addition of existing growth factors.

Accordingly, the inventive method for culturing and proliferating hematopoietic stem cells or progenitor cells can be used for the production of safe hematopoietic stem cells or progenitor cells for use in clinical transplantation, and its advanced embodiments can be used for the regeneration and proliferation for umbilical cord blood which is now freeze-stored in umbilical cord blood banks.

### Examples

Hereinafter, the present invention will be described in more detail by the following examples. It is to be understood, however, that these examples are given for illustrative purpose only and are not construed to limit the scope of the present invention.

Particularly, although only the umbilical cord blood-derived hematopoietic stem cells or progenitor cells were illustrated in the following examples, it is obvious to a person skilled in the art that even if cells derived from bone marrow, peripheral blood or non-human embryonic stem cells are used, the same or similar results can be obtained.

Moreover, although only hematopoietic stem cells showing a positive response to an antibody to umbilical cord blood-derived CD34⁺ was illustrated in the following examples, it is also possible to use hematopoietic stem cells showing a positive response to an antibody to CD133, C-Kit, CD117, CD45 or KDR, or negative responses to antibodies to CD38 and lin (Kazuta, Y et al., Stem Cells, 21:143, 2003; Yong, J.L. et al., Blood, 103:4449, 2004; Katharina, R. et al., Clin. Chim. Acta, 343:85, 2004; Cerdan, C. et al., Blood, 103:2504, 2004; Naoyuki, U. et al., J. Clin. Invest., 108:1071, 2001; Karen, E.J. et al., Cell Research, 14:268, 2004).

In addition, it is also possible to use progenitor cells showing a positive response to an antibody to Oct4, Nanog, Wnt, Stat3, alkaline phophatase or SSEA-1 (Anna, M. et al., Physiol. Rev., 85:635, 2005; Abeyta, M.J. et al., Hum. Mol. Genet., 13:601, 2004).

### Example-1: Cell collection

Umbilical cord blood was collected from full-term and preterm deliveries in Seoul National University Hospital and Samsung Cheil Hospital according to the Institutional Review Board (IRB) guidelines of Seoul National University Hospital.

The blood was collected in 250-ml standard blood collection bags (GreenCross, Korea) containing citrate-phosphate-dextrose anticoagulant (CPDA).

### Example 2: CD34⁺ cell enrichment

From isolated mononuclear cells by positive selection using an immune-magnetic system, enriched CD34⁺ cells were prepared according to the manufacturer's instruction manual (Miltenyi Biotec, Inc, Bergischm Gladbach, Germany).

"Namely, a blocking reagent and anti-CD34⁺ microbeads were added to mononuclear cells (50 x 10⁶/ml), and the mixture was reacted at 4°C for 30 minutes. The cells were passed through a plastic column in the presence of a magnet, so that the labeled cells were left to stand in the column, and the unlabeled cells were collected in a plastic tube containing RPMI-1640 (Gibco-BRL, Rockville, MD). The whole process was performed in a laminar flow hood. Then, the cells were centrifuged, re-suspended in a culture medium, and counted.

### Example 3: CD34⁺ cell quantification

After positive selection, the CD34⁺ cells were immediately identified and quantified by immunocytochemistry. For this purpose, a commercial kit (Miltenyi Biotec, Inc, Bergischm Gladbach, Germany) and a specific monoclonal antibody to a CD34⁺ antigen were used and the whole process was performed according to the manufacturer's instruction.

### Example 4: Preparation of endometrial tissue

Uterine tissues were collected from women with the normal menstrual cycle, who had a hysterectomy. Specifically, 2-3 g of uterine tissue were obtained from women in the proliferative phase (5-14 days) and the secretory phase (15-26 days), who received no hormonal treatment within 30 days. Such tissues were used for studies under the approval of the Institutional Review Board (IRB) of Samsung Cheil Hospital.

The endometrial tissues were placed in a M199/F12 medium (Gibco Life Technologies, Gaithersburg, MD, USA) containing 10% FBS, 200 units of penicillin, 0.2 mg/ml streptomycin and 0.5 µg/ml amphotericin-B) and transferred to a laboratory.

### Example 5: Isolation of endometrial stromal cells and epithelial cells

The uterine tissues transferred to the laboratory were washed with Hanks BSS to remove blood cells and various tissue residues. The isolation of epithelial cells and stromal cells was performed by a slight modification of the methods described in Satyaswaroop et al., J. Clin. Endocrinol. Metabol., 48:639, 1979 and in Julia, T. et al., Human Reprod, 16:836, 2001.

600 g of the tissues were lightly centrifuged to remove the supernatant, and the remaining tissues were placed on a 100mm dish (Coming-Costar, Cambridge, MA, USA) and finely cut into a size of 1-2 mm with a sterilized scalpel. Then, the cut tissues were placed in a M199/F12 medium containing 2 mg/ml of collagenase (Sigma, St Louis, MO, USA) and incubated in a shaking incubator at 37 °C for 2 hours and 30 minutes. The digested tissues were filtered through a 100-wire cloth sieve (140 µm size; Newark Wire Co., Newark, NJ, USA). At this time, less digested tissue masses were caught on the sieve, and the cells passed through the 100-wire cloth sieve were filtered again through a 400-wire cloth sieve (37 µm). At this time, the epithelial cells were caught on the upper side of the sieve, and the stromal cells were passed through the sieve.

The stromal cells and epithelial cells thus isolated were cultured on a 100 mm dish at 37 °C in 5% CO₂, The stromal cells were attached to the dish surface faster than the epithelial cells so that they were completely attached after one hour, whereas the epithelial cells were not attached to the dish surface even after one hour. In view of such properties, the cells were washed after one hour of the culture to isolate the epithelial cells and the stromal cells.

### Example 6: Culture of endometrial stromal cells

The stromal cells were cultured in a M199/F12 medium containing 10% FBS (Gibco Life-Technologies), insulin (0.62 µg/ml), transferrin (0.62 µg/ml), selenium (0.62 ng/ml), bovine serum albumin (125 µg/ml), linoleic acid (52.6 µg/ml), 100 units of penicillin, 0.1 mg/ml streptomycin and 0.25 µg/ml amphotericin B, on the basis of the method described in Julia, T. et al., Human Reprod, 16:836; 2001. On the other hand, the epithelial cells were cultured in a medium which was the same as the above except that it contained 2 ml/L of BPE in place of 10% FBS. The media were replaced every two days.

### Example 7: Co-culture of CD34⁺ enriched cells and primary human endometrium stromal cells

2 x 10⁵ of the stromal cells were plated on a 24-well culture flask (Nunc, Rochester, NY), and when the stromal cells reached a confluence of more than 90%, they were then seeded with CD34⁺ cells. In co-culture, the stromal cells were washed five times with PBS before the addition of the umbilical cord blood-derived CD34⁺ cells. To a monolayer of primary stromal cells cultured in a serum-free RPMI-1640 medium (Gibco-BRL, Rockville, MD) at 37 °C in 5%CO₂, 1.5 x 10⁵ of umbilical cord blood-derived CD34⁺ cells were seeded. For the comparison of proliferation rate, human breast fibroblast cells prepared by primary culture were co-cultured in the same manner as described above.

In order to compare the proliferation rate by factors secreted from the stromal cells and fibroblasts adhered in co-culture to the proliferation rate by cytokines, the media were added with each different combination of recombinant hematopoietic cytokines, i.e., 10 ng/ml of interleukin-3 (IL-3; Sigma, St Louis, MO, USA) 10 ng/ml of granulocyte-macrophage colony-stimulating factor (GM-CSF; Sigma Co., St Louis, MO, USA) and 3 U/ml of erythropoietin (EPO; Sigma, St Louis, MO, USA).

During co-culture, nonadherent/adherent hematopoietic cells were collected every day by careful pipetting. Stromal cells located below suspended cobblestone-forming hematopoietic cells were allowed to remain in the culture flask, and the total number of viable cells among the collected CD34⁺ cells was counted with a hemocytometer by trypan blue stain.

In the present invention, regardless of the type of cells produced, the new production of cells from a certain cell population was defined as cell proliferation. Thus, *in vitro* proliferation means the total number of cells produced in culture.

A CD34⁺ -enriched cell population was collected by positive immuno-magnetic microbead selection (**FIG. 1**).

The CD34⁺-enriched cells were co-cultured with endometrial stromal cells, and the co-cultured cells were treated with cytokines. Then, cell number counted at intervals in this case was compared with that in the case of culture of hematopoietic stem cells alone (see **FIGS. 2A-2D**). As a result, as shown in **FIG. 2A-2D**, the co-culture of the CD34⁺-enriched cells and the human endometrial stromal cells showed a predominant increase in the number of CD34⁺-enriched cells as compared to the culture of hematopoietic stem cells alone. Also, since FBS was not added, the CD34⁺-enriched cell population was gradually decreased starting from 3 days after co-culture. However, the proliferation pattern of the CD34⁺-enriched cells was maintained. Meanwhile, the addition of all the three cytokines (IL-3, EPO, and GM-CSF) showed an increase in cell number as compared to the addition of each of the cytokines. The case of treatment with IL-3 among the three cytokines showed the greatest increase in cell number as compared to the case of treatment with each of the other cytokines. However, the treatment with the cytokines did not show a significant increase in cell number as compared to the case of co-culture.

To compare the effect of the endometrial stromal cells in co-culture with the effect of other fibroblasts, an experiment was carried out using a test group containing human breast fibroblasts in place of the endometrial stromal cells. For this purpose, the CD34⁺-enriched cells were co-cultured with breast fibroblasts, and the co-cultured cells were treated with cytokine (IL-3, LIF: leukemia inhibitory factor). Then, cell number counted at intervals in this case was compared with that in the case of the co-culture of the CD34⁺-enriched cells and the endometrial stromal cells and that in the case of culture of hematopoietic stem cells alone (see **FIGS. 3A** and **3B****).** As a result, as shown in **FIGS. 3A** and **3B****,** the co-culture of the CD34⁺-enriched cells and the breast fibroblasts showed an increase in cell number as compared to the culture of the hematopoietic stem cells alone, but a reduction in cell number as compared to the case of the co-culture with the endometrial stromal cells. Also, the treatment with cytokine (IL-3) did not show a significant increase in the cell number as compared to the co-culture (see **FIGS. 2A-2D** and **FIGS. 3A** and **3B****).**

### INDUSTRIAL APPLICABILITY

As described above in detail, the present invention provides the method for culturing and proliferating the hematopoietic stem cells or the progenitor cells, in which the CD34⁺ hematopoietic stem cells or the progenitor cells are co-cultured with the human endometrial stromal cells or epithelial cells in a serum-free or serum medium. According to the present invention, hematopoietic stem cells or progenitor cells, which are safe for transplantation, can be proliferated in large amounts in a short period of time. Thus, the present invention will be useful for the treatment of patients with intractable or incurable diseases, who are in need of the clinical transplantation of hematopoietic stem cells or progenitor cells.

## Claims

1. A method for culturing and proliferating hematopoietic stem cells or progenitor cells, the method comprising the steps of:
(a) culturing hematopoietic stem cells or progenitor cells derived from umbilical cord blood, marrow, peripheral blood or non-human embryonic stem cells;
(b) culturing endometrial stromal cells or epithelial cells derived from uterine tissue; and
(c) co-culturing the hematopoietic stem cells or progenitor cells cultured in the step (a) and the endometrial stromal cells or epithelial cells cultured in the step (b).

2. The method according to claim 1, wherein the hematopoietic stem cells or progenitor cells are obtained by the steps of:
(a) monolayer culturing, suspension culturing or co-culturing mononuclear or progenitor cells derived from umbilical cord blood, marrow, peripheral blood or non-human embryonic stem cells;
(b) reacting the monolayer-, suspension- or co-cultured cells with an antibody to a hematopoietic stem cell- or progenitor cell-specific antigen; and
(c) isolating the cells bound to the antibody by means of a cell sorter or a column.

3. The method according to claim 2, wherein the antibody to the hematopoietic stem cell-specific antigen is an antibody to CD34⁺, CD38⁻, Thyl.1⁺, lin⁻, CD45⁺, or KDR⁺.

4. The method according to claim 2, wherein the antibody to the progenitor cell-specific antigen is an antibody to Oct4, Nanog, Wnt, Stat3, Alkaline phophatase, or SSEA-1.

5. The method according to claim 2, wherein the cell sorter is a magnetic cell sorter and the column is a magnetic column.

6. The method according to claim 1, wherein the endometrial stromal cells or epithelial cells are obtained by the steps of:
(a) placing and culturing finely cut uterine tissue in a cell culture medium containing collagenase;
(b) passing the cultured cells through a cloth sieve with a pore size of 30-50 um, and collecting the epithelial cells caught on the cloth sieve; and
(c) culturing the cells passed through the sieve in the step (b) on a cell culture dish and then isolating the stromal cells adhered to the cell culture dish by washing the cell culture dish with the culture broth.

7. The method according to claim 1, wherein the co-culture in the step (c) is performed in a serum-free or serum medium.

8. The method according to claim 1, wherein the co-culture in the step (c) is performed for 1 - 120 hours.

## Patentansprüche

1. Verfahren zur Kultivierung und Proliferation hämatopoetischer Stammzellen oder Vorläuferzellen, umfassend die Stufen:
(a) Kultivierung hämatopoetischer Stammzellen oder Vorläuferzellen, abgeleitet von Nabelschnurblut, Knochenmark, peripherem Blut oder nicht-humanen embryonalen Stammzellen;
(b) Kultivierung endometrialer Bindegewebszellen oder Epithelzellen, abgeleitet von Uterusgewebe und
(c) Co-Kultivierung hämatopoetischer Stammzellen oder Vorläuferzellen, kultiviert in Schritt (a) und die endometrialer Bindegewebszellen oder Epithelzellen, kultiviert in Schritt (b).

2. Verfahren gemäß Anspruch 1, wobei die hämatopoetischen Stammzellen oder die Vorläuferzellen durch diese Stufen gewonnen werden:
(a) Monolayerkultivierung, Suspensionskultivierung oder Co-Kultivierung einkerniger oder Vorläuferzellen, abgeleitet von Nabelschnurblut, Knochenmark, peripherem Blut oder von nicht-humanen emryonalen Stammzellen;
(b) Reaktion der Monolayer-, Suspensions- oder cokultivierten Zellen mit einem Antikörper gegen ein hämatopoetisches stammzell- oder vorläuferzellspezifisches Antigen und
(c) Isolierung der antikörpergebundenen Zellen mittels eines Zellsorters oder einer Säule.

3. Verfahren gemäß Anspruch 2, wobei der Antikörper gegen das hämatopoetische stammzellspezifische Antigen ein Antikörper gegen CD34⁺, CD38⁻, Thyl.1⁺, lin⁻, CD45⁺ oder KDR⁺ ist.

4. Verfahren gemäß Anspruch 2, wobei der Antikörper gegen das vorläuferzellspezifische Antigen ein Antikörper gegen Oct4, Nanog, Wnt, Stat3, alkalische Phosphatase oder SSEA-1 ist.

5. Verfahren gemäß Anspruch 2, wobei der Zellsortierer ein magnetischer Zellsortierer ist und die Säule eine magnetische Säule ist.

6. Verfahren gemäß Anspruch 1, wobei die endometrialen Bindegewebszellen oder die Epithelzellen durch die Stufen gewonnen werden:
(a) Platzierung und Kultivierung fein geschnittenen Uterusgewebes in Kollagenase enthaltendes beziehungsweise enthaltendem Zellkulturmedium;
(b) Passierung der kultivierten Zellen durch ein Gewebesieb mit einer Porengröße von 30 bis 50µm und das Auffangen der im Gewebesieb zurückgehaltenen Epithelzellen und
(c) Kultivierung der in Stufe (b) durch ein Sieb passierten Zellen in einer Zellkulturschale und die anschließende Isolierung der Bindegewebszellen welche an die Zellkulturschale gebunden haben durch Waschen der Zellkulturschale mit Kulturmedium.

7. Ein Verfahren gemäß Anspruch 1, wobei die Co-Kultivierung in Stufe (c) in serumfreiem beziehungsweise serumhaltigem Medium durchgeführt wird.

8. Verfahren gemäß Anspruch 1, wobei die Co-Kultivierung in Stufe (c) für 1-120 Stunden durchgeführt wird.

## Revendications

1. Procédé de culture et de prolifération de cellules souches hématopoïétiques ou de cellules progénitrices, le procédé comprenant les étapes de :
(a) culture des cellules souches hématopoïétiques ou des cellules progénitrices dérivées de sang de cordon ombilical, de moelle, de sang périphérique ou de cellules souches embryonnaires non humaines ;
(b) culture des cellules stromales endométriales ou des cellules épithéliales dérivées de tissu utérin ; et
(c) co-culture des cellules souches hématopoïétiques ou des cellules progénitrices cultivées à l'étape (a) et des cellules stromales endométriales ou des cellules épithéliales cultivées à l'étape (b).

2. Procédé selon la revendication 1, dans lequel les cellules souches hématopoïétiques ou les cellules progénitrices sont obtenues par les étapes de :
(a) culture en monocouche, culture en suspension ou co-culture de cellules mononucléaires ou progénitrices dérivées de sang de cordon ombilical, de moelle, de sang périphérique ou de cellules souches embryonnaires non humaines ;
(b) réaction des cellules en monocouche, en suspension ou co-cultivées avec un anticorps contre un antigène spécifique d'une cellule souche hématopoïétique ou d'une cellule progénitrice ; et
(c) isolement des cellules liées à l'anticorps au moyen d'un trieur de cellules ou d'une colonne.

3. Procédé selon la revendication 2, dans lequel l'anticorps contre l'antigène spécifique d'une cellule souche hématopoïétique est un anticorps CD34⁺, CD38⁻, Thyl.1⁺, lin⁻, CD45⁺ ou KDR⁺.

4. Procédé selon la revendication 2, dans lequel l'anticorps contre l'antigène spécifique de la cellule progénitrice est un anticorps de Oct4, Nanog, Wnt, Stat3, la phosphatase alcaline ou SSEA-1.

5. Procédé selon la revendication 2, dans lequel le trieur de cellules est un trieur de cellules magnétique et la colonne est une colonne magnétique.

6. Procédé selon la revendication 1, dans lequel les cellules stromales endométriales ou les cellules épithéliales sont obtenues par les étapes de :
(a) placement et culture d'un tissu utérin finement coupé dans un milieu de culture cellulaire contenant de la collagénase ;
(b) passage des cellules cultivées dans un tamis en toile présentant une taille de pores de 30 à 50 µm et recueil des cellules épithéliales retenues sur le tamis en toile ; et
(c) culture des cellules qui ont traversé le tamis à l'étape (b) sur une boîte de culture cellulaire puis isolement des cellules stromales adhérant à la boîte de culture cellulaire par lavage de la boîte de culture cellulaire avec le bouillon de culture.

7. Procédé selon la revendication 1, dans lequel la co-culture à l'étape (c) est réalisée dans un milieu sans sérum ou avec sérum.

8. Procédé selon la revendication 1, dans lequel la co-culture à l'étape (c) est réalisée pendant 1 à 120 heures.
